# EUROPÄISCHE PATENTANMELDUNG

(11) **EP 4 148 109 A1**
(43) Veröffentlichungstag der Anmeldung: **15.03.2023**
(21) Anmeldenummer: 22188271.5
(22) Anmeldetag: 02.08.2022
(51) Int. Cl.: C10L 3/08, C01B 3/04, C01B 3/34, C01B 3/48, C07C 1/12, C07C 9/04, C25B 1/04, F25J 1/00, F25J 3/06

(54) **ENERGIE- UND WASSERSTOFFLOGISTIK**

(30) Priorität: 10.09.2021 DE 102021123556
(71) Anmelder: MAN Energy Solutions SE, 86153 Augsburg (DE)
(72) Erfinder: Bank, Rolf, 94469 Deggendorf (DE); Anger, Norbert, 4020 Linz (AT)

(57) **Zusammenfassung**

Bei einem Verfahren zum Transport von Wasserstoff in Form von flüssigem Methan, mit den folgenden Schritten: a) Erzeugen von Elektrizität in Anlagen zur Nutzung von erneuerbaren Energien, insbesondere von Wind, Sonne, Biomasse oder Geothermie; b) Verwenden der im Schritt a) erzeugten Elektrizität, um Wasser in Wasserstoff und Sauerstoff aufzuspalten; c) Bereitstellen von Kohlendioxid; d) Zuführen des Wasserstoffs aus Schritt b) und des Kohlendioxids aus Schritt c) in ein Reaktorsystem zur Herstellung von Methan, wobei dieses Reaktorsystem einen mit Siedewasser gekühlten katalytischen Reaktor aufweist; e) Verflüssigen des so hergestellten Methans; f) Transportieren des verflüssigten Methans zu einem entfernt gelegenen Verbrauchsort; g) Nutzung des verflüssigten Methans am Verbrauchsort unter Erzeugen von Kohlendioxid; h) Abscheiden dieses Kohlendioxids; wobei Schritt c) den Rücktransport von Kohlendioxid aus Schritt h) umfasst; umfasst Schritt g) folgenden Schritt: g1) am Verbrauchsort wird das Methan einer Dampfreformierung zur Herstellung von Wasserstoff unterzogen, wobei Kohlendioxid entsteht; und umfasst Schritt c) folgenden Schritt: c1) zumindest ein Teil des bei der Dampfreformierung entstandenen Kohlendioxids wird zum Reaktorsystem zur Herstellung von Methan zurücktransportiert.

## Beschreibung

Die Erfindung bezieht sich auf ein Verfahren nach dem Oberbegriff von Anspruch 1.

Mit solchen Verfahren wird Energie in Wasserstoff umgewandelt und dieser in einen anderen chemischen Stoff überführt, der geringere Transportkosten als der Transport von Wasserstoff verursacht. Hierdurch kann relativ kostengünstig Energie aus Regionen mit überschüssiger Energie, beispielsweise Sonnenenergie aus dem Sonnengürtel der Erde oder Windenergie aus windreichen Regionen wie aus dem Süden von Südamerika, zu Verbrauchern gebracht werden, die sehr weit entfernt gelegen sind, insbesondere auf anderen Kontinenten. Hierbei ist es bekannt, den durch die Umwandlung der Energie erzeugten Wasserstoff durch eine Hydrierreaktion an einen flüssigen, organischen Wasserstoffträger (LOHC, Liquid Organic Hydrogen Carrier) zu binden. Dadurch kann der Wasserstoff wie ein Rohöl transportiert werden. Am Bestimmungsort wird durch eine weitere chemische Reaktion (Dehydrierung vom LOHC) der Wasserstoff freigesetzt und steht zur Verfügung.

In AU 2011101411 A4 ist ein gattungsgemäßes Verfahren beschrieben, insbesondere eine Methode zur Sequestrierung von Kohlendioxid durch die Herstellung und den Export von erneuerbarem Flüssig-Erdgas (LNG, liquid natural gas), die die folgenden Schritte umfasst: 1. Erzeugung von Wasserstoff aus erneuerbarer Energie; 2. Einspeisung dieses Wasserstoffs in einen Sabatier-Reaktor zusammen mit Kohlendioxid aus einer Kohlendioxidquelle eines inländischen oder überseeischen Emittenten, um Methan und Wasser zu erzeugen; 3. Transport dieses Methans zu einer australischen LNG-Anlage, von der aus Schiffe es zu LNG-Kunden in Übersee exportieren können.

Aus US 2011/0064647 A1 ist eine Vorrichtung und ein Verfahren zur Speicherung und zum Transport von Wasserstoff unter Verwendung von Kohlendioxid als Speichermedium bekannt. Ein Elektrolyseur verwendet Energie aus erneuerbaren Quellen zur Bereitstellung von Wasserstoff durch Dissoziation von Wasser. Ein Reaktor bildet ein Produkt, vorzugsweise Methan, durch Reaktion von Wasserstoff und Kohlendioxid. Das Produkt wird zu einem Verbrauchsort oder dem Speicherort transportiert. Eine Speichervorrichtung kann verwendet werden, um abgeschiedenes Kohlendioxid zu speichern, das beim Verbrauch des Produkts entsteht. Dieses abgeschiedene Kohlendioxid wird zum Reaktorstandort zurück transportiert, um dort mit dem aus einer Wasserstoffquelle bereitgestellten Wasserstoff zu reagieren. Somit wird ein Kohlendioxidkreislauf verwendet, um Wasserstoff effizient zu transportieren und zu speichern.

DE 2940334 A1 offenbart ein Verfahren zum Methanisieren eines im Wesentlichen Wasserstoff und Oxide des Kohlenstoffs enthaltenden Synthesegases durch katalytische Umsetzung bei erhöhten Temperaturen und Drücken. Die Methanisierung wird unter Wasserstoffüberschuss durchgeführt, wobei der überschüssige Wasserstoff aus dem Produktstrom abgetrennt und in die Methanisierung zurückgeführt wird.

Die Aufgabe der vorliegenden Erfindung liegt darin, ein Verfahren der eingangs genannten Art so zu verbessern, dass die am Verbrauchsort zur Verfügung stehende Wasserstoffmenge erhöht wird.

Erfindungsgemäß wird diese Aufgabe durch die Gemeinsamkeit aller Merkmale des Anspruchs 1 gelöst.

Die erfindungsgemäßen Maßnahmen ermöglichen, dass zum einen im Trägermaterial eine große Wasserstoffmenge (25% Wasserstoff im Vergleich zu 6,3% beim LOHC-Konzept) transportiert wird und außerdem am Verbrauchsort durch eine Dampfreformierung des Methans und eine sich eventuell anschließende Wassergas-Shift-Reaktion die verfügbare Menge an Wasserstoff verdoppelt wird. Das entstehende CO2 kann abgetrennt und zurücktransportiert werden, so dass das CO2 im Kreislauf geführt wird. Durch die Kombination an sich bekannter Prozesse in der Reihenfolge
- Methanisierung und Methanverflüssigung am energiereichen Ort einerseits,
- dann Transport (LNG-Transportmittel, z. B. LNG-Tanker bei Transport über Seeweg) und
- Wasserdampfreformierung einschließlich einer möglichen Wassergas-Shift-Reaktion am Verbrauchsort des Wasserstoffs andererseits
sind am Verbrauchsort pro Kilogramm transportierten Trägermaterials 0,5 kg Wasserstoff (also 50%) verfügbar.

Dabei geht die Erfindung von der Erkenntnis aus, dass bei einem Vergleich der verschiedenen Transportmöglichkeiten mittels eines Wasserstoffträgers (LOHC, Ammoniak, Methanol oder LNG) der Wasserstoffanteil bei LNG mit 25 Massen-% am höchsten ist. Wird das LNG strombasiert hergestellt, so spricht man auch von "eLNG". Bei LOHC beträgt der Wasserstoffanteil 6,2 Massen-%, bei Ammoniak 17,6 Massen-% und bei Methanol 12,5 Massen-%. Die Energiedichte, die mittels eLNG aus der energiereichen Region transportiert wird, liegt mit 15,5 kWh/kg(eLNG) am höchsten. LOHC liefert über die Verbrennung des transportierten Wasserstoffs 2,1 kWh/ kg(LOHC). Ammoniak besitzt eine Energiedichte von 6,25 kWh/kg(Ammoniak) und Methanol liegt mit 6,30 kWh/kg(Methanol) auf vergleichbarem Niveau wie Ammoniak. Daraus ergibt sich, dass in jedem Fall der Transport von eLNG am effektivsten ist, unabhängig davon, ob der Transport von Energie, Wasserstoff oder beide im Vordergrund stehen.

Ein erfindungsgemäßer Prozess überführt also den Ausgangsstoff "Wasserstoff" über den Zwischen-Energieträger "Methan" wieder zurück in den Ausgangsstoff. Dabei nimmt dieser Zwischen-Energieträger weniger Volumen ein und erfordert weniger Aufwand zum Kühlen und zum Verdichten als andere Zwischen-Energieträger. Somit kann bei gleichem Transportvolumen eine größere Energiemenge bei höherer Temperatur und niedrigerem Druck transportiert werden.

Ferner kann ein erfindungsgemäßer Prozess flexibel genutzt werden. Er ermöglicht alternativ die Nutzung des mit Hilfe des Zwischen-Energieträgers transportierten Wasserstoffs oder des Zwischen-Energieträgers selbst. CO2 wird in der Gesamtbetrachtung in einem weitgehend geschlossenen Kreislauf gefahren.

Für alle Einzelprozesse des Gesamtverfahrens kommen kommerziell ausgereifte Einrichtungen zum Einsatz, so dass das erfindungsgemäße Verfahren ohne Verzögerung umgesetzt werden kann.

Es ist fraglich, ob sich der Transport von flüssigem Wasserstoff auf Grund der Probleme von Energieverlusten und des Sicherheitsaufwandes großtechnisch etablieren wird. Der erfindungsgemäße Prozess überwindet diese Probleme.

Bevorzugt umfasst Schritt h) einen Schritt h1), gemäß dem im Schritt g1) das durch die Dampfreformierung entstandene Reaktionsgas durch Kühlung verflüssigt und während des Abkühlvorgangs das verflüssigte Kohlendioxid vom gasförmigen Wasserstoff abgetrennt wird, und wird im Schritt c1) das abgetrennte Kohlendioxid mittels eines CO2-Transportmittels zurücktransportiert. Mit diesen Maßnahmen wird der Wasserstoff effektiv und kostengünstig vom Kohlendioxid getrennt und kann das nunmehr verflüssigte Kohlendioxid ebenfalls kostengünstig dem Reaktorsystem zur Herstellung von Methan wieder zugeführt werden.

Vorteilhafterweise wird durch die Schritte d) bis f), g1), h) und c1) ein weitgehend geschlossener CO2-Kreislauf gebildet. CO2-Emissionen werden somit minimiert.

In einer günstigen Ausgestaltung der Erfindung wird im Schritt d) die Methanisierung mit einem Überschuss von Wasserstoff in Bezug auf die Umsetzung des Kohlendioxids von unter 10 Vol-%, jedoch mit mindestens 0,3 Vol-% betrieben. Das Verfahren wird bevorzugt bei einem Betriebsdruck von mindestens 20 bar am Reaktoreingang betrieben. Dabei beträgt der Wasserstoffüberschuss bevorzugt mehr als 1,0 Vol-% und besonders bevorzugt mehr als 1,5 Vol-%. Durch den Betrieb der Methanisierung mit H2-Überschuss (Verhältnis von H2:CO2 >4 im Reaktorzulauf) wird der CO2-Umsatz maximiert. Im Produktgas befinden sich nur noch kleine bis vernachlässigbare Mengen CO2, die sich an den Kühlflächen der Verflüssigungsanlage als Feststoff niederschlagen und von Zeit zu Zeit durch Abschmelzen entfernt werden.

In vorteilhafter Ausgestaltung der Erfindung wird im Schritt e) der überschüssige Wasserstoff in der Gasphase vom flüssigen Methan abgetrennt und wird im Schritt d) dieser überschüssige Wasserstoff zum Reaktorsystem wieder zurückgeführt. Durch Rückführung des überschüssigen H2 in den Eingang des Methanisierungsreaktors geht kein H2 verloren.

Vorzugsweise wird im Schritt b) die in Schritt a) erzeugte Elektrizität zum Betreiben einer Elektrolyseanlage verwendet. Eine großtechnische Umsetzung der Spaltung von Wasser ist damit ohne weiteres möglich.

In einer günstigen Weiterentwicklung der Erfindung umfasst Schritt c) das Sammeln von Kohlendioxid aus einer Emissionsquelle, insbesondere aus einem mit Methan betriebenen Kraftwerk, einer Biomasse-Energieanlage oder einer Kohlendioxid ausstoßenden Industrieanlage. Damit ist sichergestellt, dass das Kohlendioxid umweltfreundlich bereitgestellt wird.

Die Erfindung wird an Hand der einzigen Figur beispielshalber noch näher erläutert:

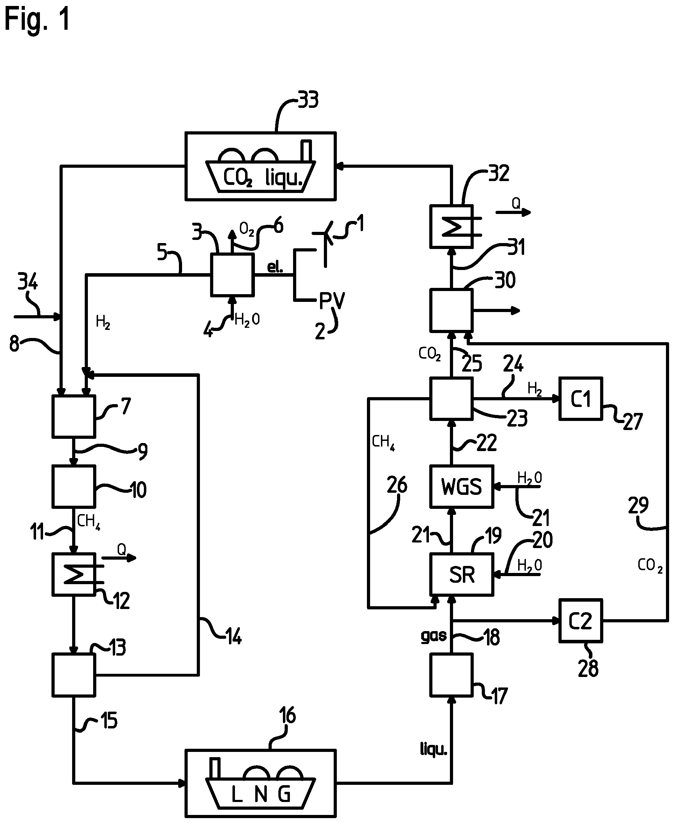

- Figur 1: zeigt ein Ausführungsbeispiel des erfindungsgemäßen Verfahrens zum Transport von Wasserstoff in Form von flüssigem Methan.

Den Ausgangspunkt des Verfahrens bildet die Erzeugung von elektrischer Energie durch erneu-erbare Energiequellen, beispielweise aus Windenergie 1 oder aus Sonnenenergie 2. Diese elektrische Energie wird - z. B. in einem Elektrolyseur 3 - zur Spaltung von Wasser 4 in Wasserstoff 5 und Sauerstoff 6 genutzt. Der Sauerstoff 6 wird im einfachsten Fall in die Atmosphäre entlassen. Mit Vorteil kann er jedoch in industriellen Prozessen verwendet werden oder zur Unterstützung der biologischen Abwasserreinigung in Kläranlagen. Der erzeugte Wasserstoff 5 wird in einem Mischer 7 mit einem CO2-Strom 8 zu einem Reaktionsgas 9 zusammengeführt. Das Mischungsverhältnis wird dabei vorzugsweise so eingestellt, dass es Wasserstoff im überstöchiometrischen Verhältnis enthält. In einem Methanisierungsreaktor 10 wird das Reaktionsgas 9 zu Methan 11 umgesetzt.

Das umgesetzte Reaktionsgas wird in einem Kühler 12 gekühlt und verflüssigt. Kondensierbare Reaktionsprodukte wie Wasser werden in einem Abscheider 13 abgetrennt und nicht umgesetzte Reaktionsgaskomponenten werden über eine Rücklaufleitung 14 bevorzugt über den Mischer 7 zum Eintritt in den Methanisierungsreaktor 10 wieder zurückgeführt. Das so verflüssigte Methan 15, auch als LNG (liquid natural gas) bezeichnet, wird in ein speziell ausgerüstetes Transportmittel, bei einem Transport über See in einen speziell ausgerüsteten Tanker, 16 gefördert und mit diesem an einen örtlich weiter entfernten Verbrauchsort transportiert.

Dort angekommen, wird es ggf. in hier nicht dargestellten Tanks zwischengelagert. In einem Verdampfer 17 wird es wieder in gasförmiges Methan 18 überführt. Das Methan 18 wird in einem Dampfreformer (SR, steam reformer) 19 mit Wasserdampf 20 zu Synthesegas 21, bestehend aus Kohlendioxid und Wasserstoff, umgesetzt. In einer bevorzugten Ausführungsform wird durch Zufuhr von weiterem Wasserdampf (WGS, water gas shift) 21 das Reaktionsgas schließlich zu einem Produktgas 22 umgesetzt. In einem Abscheider 23 wird dieses gekühlt in seine Hauptbestandteile Wasserstoff (H2, gasförmig) 24 und Kohlendioxid (CO2, flüssig) 25 getrennt. Nicht umgesetztes Methan 26 wird in den Dampfreformer 19 wieder zurückgeführt. Der erzeugte Wasserstoff 24 wird einem Wasserstoffnutzer (C1) 27 zugeführt. Ein anderer Nutzungsweg des aus dem Verdampfer 17 strömenden gasförmigen Methans 18 ist die konventionelle direkte Nutzung durch einen Verbraucher (C2) 28. Dieser kann z.B. ein Gaskraftwerk sein, ein Industriebetrieb oder eine Heizungsanlage. In der Regel besteht diese Nutzung aus Verbrennungsprozessen unter Bildung von CO2 29. Bevorzugt wird dieses aus dem Abgas abgeschieden und in gleicher Weise wie das in der bevorzugten Ausführungsform entstandene CO2 25 einer Reinigungsstufe 30 zugeführt. Das so gereinigte CO2 31 wird in einem Kühler 32 verflüssigt und in einem zu Transportmittel 16 baugleichen oder ähnlichem Transportmittel 33 zu dem Mischer 7 der Methanisierungsanlage zurückgeführt. Es ist ebenfalls möglich, dass das Transportmittel 16 als Transportmittel 33 für das CO2 verwendet wird.

Das CO2 31 aus den Verbrauchsprozessen wird weitgehend zurückgewonnen und im Kreislauf geführt. Der Begriff "weitgehend" ist dabei so zu verstehen, dass zwar so viel wie möglich CO2 zurückgewonnen wird. Wie bei jedem Rückgewinnungsprozess gibt es jedoch Verluste, die aus technischen und wirtschaftlichen Gründen nicht ganz vermieden werden können. Die entstandenen Verluste müssen durch andere CO2-Quellen ausgeglichen werden. Der daraus stammende CO2-Ergänzungsstrom 34 wird zusammen mit dem CO2-Strom 8 dem Mischer 7 zugeführt.

Die für die verschiedenen Teilprozesse erforderlichen Fördereinrichtungen sind dem Fachmann bekannt. Eine detaillierte Darstellung erfolgt daher hier nicht. Im Rahmen der Erfindung liegen weiterhin Modifikationen einzelner Verfahrensschritte, wie beispielsweise das Vertauschen von Reinigungs- und Kühlstufen. Die anlageninterne Wärmenutzung von Wärme erzeugenden und Wärme nutzenden Prozessen ist ebenfalls dem Fachmann bekannt und wird hier nicht weiter behandelt.

## Patentansprüche

1. Verfahren zum Transport von Wasserstoff in Form von flüssigem Methan, mit den folgenden Schritten:
a) Erzeugen von Elektrizität in Anlagen zur Nutzung von erneuerbaren Energien, insbesondere von Wind (1), Sonne (2), Biomasse oder Geothermie;
b) Verwenden der im Schritt a) erzeugten Elektrizität, um Wasser (4) in Wasserstoff (5) und Sauerstoff (6) aufzuspalten;
c) Bereitstellen von Kohlendioxid (8);
d) Zuführen des Wasserstoffs (5) aus Schritt b) und des Kohlendioxids (8) aus Schritt c) in ein Reaktorsystem zur Herstellung von Methan (11), wobei dieses Reaktorsystem einen mit Siedewasser gekühlten katalytischen Reaktor (10) aufweist;
e) Verflüssigen (12) des so hergestellten Methans (11);
f) Transportieren (16) des verflüssigten Methans (15) zu einem entfernt gelegenen Verbrauchsort;
g) Nutzung des verflüssigten Methans (15) am Verbrauchsort unter Erzeugen von Kohlendioxid (25);
h) Abscheiden (23) dieses Kohlendioxids (25);
wobei Schritt c) den Rücktransport (33) von Kohlendioxid (25) aus Schritt h) umfasst;
**dadurch gekennzeichnet, dass**
Schritt g) folgenden Schritt umfasst:
g1) am Verbrauchsort wird das Methan (15) einer Dampfreformierung (19) zur Herstellung von Wasserstoff (24) unterzogen, wobei Kohlendioxid (25) entsteht; und
Schritt c) folgenden Schritt umfasst:
c1) zumindest ein Teil des bei der Dampfreformierung (19) entstandenen Kohlendioxids (25) wird zum Reaktorsystem zur Herstellung von Methan (11) zurücktransportiert (33).

2. Verfahren nach Anspruch 1, **dadurch gekennzeichnet, dass**
Schritt h) folgenden Schritt umfasst:
h1) im Schritt g1) wird das durch die Dampfreformierung (19) entstandene Reaktionsgas durch Kühlung (23) verflüssigt und während des Abkühlvorgangs wird das verflüssigte Kohlendioxid (25) vom gasförmigen Wasserstoff (24) abgetrennt.
und dass im Schritt c1) das abgetrennte Kohlendioxid (25) mittels eines CO2-Transportmittels (33) in das Reaktorsystem gemäß Schritt d) zurücktransportiert wird.

3. Verfahren nach einem der vorangehenden Ansprüche, **dadurch gekennzeichnet, dass** durch die Schritte d) bis f), g1), h) und c1) ein weitgehend geschlossener CO2-Kreislauf gebildet wird.

4. Verfahren nach einem der vorangehenden Ansprüche, **dadurch gekennzeichnet, dass** im Schritt d) die Methanisierung mit einem Überschuss von Wasserstoff (5) in Bezug auf die Umsetzung des Kohlendioxids (8) von unter 10 Vol-%, jedoch mit mindestens 0,3 Vol-% betrieben wird.

5. Verfahren nach Anspruch 4, **dadurch gekennzeichnet, dass**
der Wasserstoffüberschuss mehr als 1,0 Vol-% beträgt.

6. Verfahren nach Anspruch 5, **dadurch gekennzeichnet, dass**
der Wasserstoffüberschuss mehr als 1,5 Vol-% beträgt.

7. Verfahren nach einem der vorangehenden Ansprüche, **dadurch gekennzeichnet, dass**
im Schritt e) der überschüssige Wasserstoff in der Gasphase vom flüssigen Methan (15) abgetrennt wird und
im Schritt d) dieser überschüssige Wasserstoff zum Reaktorsystem wieder zurückgeführt wird.

8. Verfahren nach einem der vorangehenden Ansprüche, **dadurch gekennzeichnet, dass** im Schritt b) die in Schritt a) erzeugte Elektrizität zum Betreiben einer Elektrolyseanlage (3) verwendet wird.

9. Verfahren nach einem der vorangehenden Ansprüche, **dadurch gekennzeichnet, dass** Schritt c) das Sammeln von Kohlendioxid (8) aus einer Emissionsquelle, insbesondere aus einem mit Methan betriebenen Kraftwerk, einer Biomasse-Energieanlage oder einer Kohlendioxid ausstoßenden Industrieanlage, umfasst.
